(19) Europäisches Patentamt
European Patent Office
Office européen des brevets

(11) Publication number: **0 481 320 A1**

(12) **EUROPEAN PATENT APPLICATION**

(21) Application number: **91117035.5**

(22) Date of filing: **05.10.91**

(51) Int. Cl.[5]: **C07D 473/00**, C07D 239/54, C07D 239/46, C07C 223/04

(30) Priority: **09.10.90 JP 269530/90**

(43) Date of publication of application: **22.04.92 Bulletin 92/17**

(84) Designated Contracting States: **CH DE ES FR GB IT LI NL SE**

(71) Applicant: **NIPPON KAYAKU KABUSHIKI KAISHA**
**11-2, Fujimi 1-chome Chiyoda-ku**
**Tokyo 102(JP)**

(72) Inventor: **Nagai, Masashi**
**27-6, Egota-4-chome**
**Nakano-ku, Tokyo(JP)**
Inventor: **Sagawa, Yukihiro**
**1039, Kamiochiai**
**Yono-shi(JP)**
Inventor: **Ikeda, Ryuji**
**1039, Kamiochiai**
**Yono-shi(JP)**
Inventor: **Shiozawa, Akira**
**22-7, Horisaki**
**Omiya-shi(JP)**

(74) Representative: **Türk, Gille, Hrabal**
**Brucknerstrasse 20**
**W-4000 Düsseldorf 13(DE)**

(54) **Novel cyclobutane derivatives.**

(57) Cyclobutane derivatives represented by the formula (1)

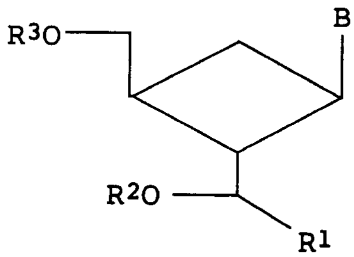

(1)

wherein B is a nucleic acid base derivative, $R^1$ denotes a lower alkyl group of 1 to 6 carbon atoms, and $R^2$ and $R^3$ each independently denote hydrogen atom or a protecting group for hydroxyl, and physiologically acceptable salts thereof. These compounds are expectedly useful as medicinal agents such as antiviral agent, carcinostatic agent and the like.

EP 0 481 320 A1

FIELD OF THE INVENTION

The present invention relates to novel cyclobutane derivatives expectedly useful as medicinal agents such as an antiviral agent, carcinostatic agent and the like.

BACKGROUND OF THE INVENTION

Many of the nucleic acid-related substances are known to have an antiviral activity or a carcinostatic activity, and some of them are in clinical use as useful medicinal agents. For example, known antiviral agents of this kind include vidarabine [M. Privat de Garilhe and J. de Rubber, C.R. Acad. Soc. D (Paris), 259, 2725 (1964)), acyclovir [G.B. Elion et al., Proc. Natl. Acad. Sci. USA, 74, 5716 (1977)], azidothymidine [H. Mitsuya et al., Proc. Natl. Acad. Sci. USA, 82, 7096 (1985)] and others. As to carcinostatic agents of this kind, there are known 5-fluorouracil, cytosine arabinoside and others.

Further, nucleic acid derivatives are also known which are represented by the following formula and have an antiviral activity [J.A., Vol. XLII (No. 12), 1989, pp 1854-1859; Antimicrob. Agents Chemother., vol. 32 (No. 7), 1988, pp 1053-1056]

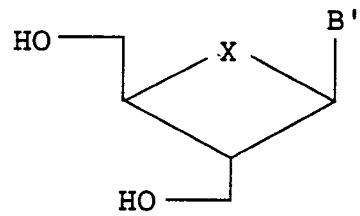

wherein B' is adenine or guanine and X is oxygen atom or carbon atom.

The object of the present invention is to provide novel cyclobutane derivatives expectedly useful for their strong antiviral activity.

SUMMARY OF THE INVENTION

The present invention relates to novel cyclobutane derivatives represented by the following formula (1), physiologically acceptable salts thereof, intermediates therefor, and processes for the production thereof:

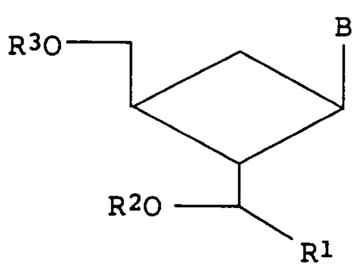

(1)

wherein B is a nucleic acid base derivative, $R^1$ denotes a lower alkyl group of 1 to 6 carbon atoms, and $R^2$ and $R^3$ each independently denote hydrogen atom or a protecting group for hydroxyl.

DETAILED DESCRIPTION OF THE INVENTION

Examples of the nucleic acid base derivative represented by B in the formula (1) include, for example, purine bases, pyrimidine bases and those bases protected by a protecting group. As examples of said purine bases, compounds represented by the following formulas can be referred to:

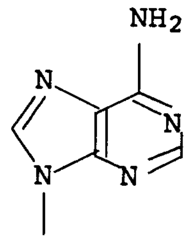
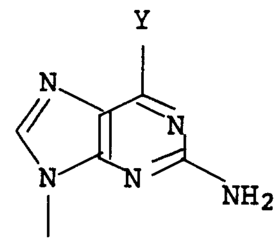
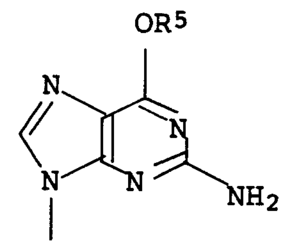

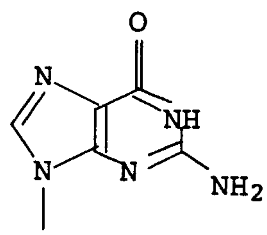
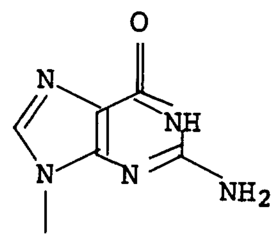
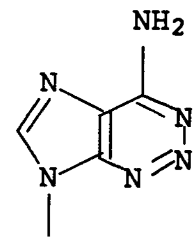

wherein Y denotes hydrogen atom, an amino group or a halogen atom such as chlorine, bromine, fluorine and the like, and $R^5$ denotes an alkyl group of 1-20 carbon atoms such as methyl, ethyl and the like, a substituted or unsubstituted benzyl group such as benzyl and 4-methoxybenzl, $(C_1-C_4)$alkoxy$(C_1-C_4)$alkyl group such as methoxyethyl and the like, or an aryl group such as phenyl.

As examples of said pyrimidine bases, compounds represented by the following formulas can be referred to:

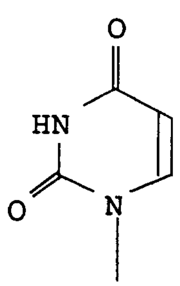
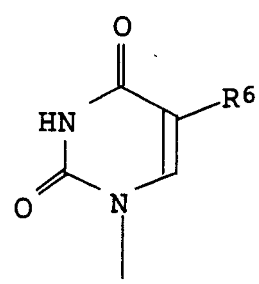
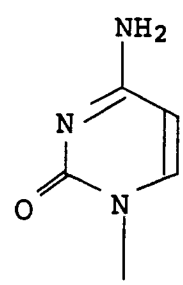

wherein $R^6$ denotes hydrogen atom, a lower alkyl group of 1 to 4 carbon atoms such as methyl, ethyl, butyl and the like, a 2-halogen substituted vinyl group such as 2-bromovinyl, 2-iodovinyl and the like, or a halogen atom such as iodine, bromine, chlorine and fluorine.

As the protecting group for hydroxyl in $R^2$ and $R^3$, all the groups which are generally used as a protecting group can be used with no particular restriction, including ester type protecting groups, ether type protecting groups and the like.

As examples of the ester type protecting group, mention may be made of acyl groups such as acetyl, benzoyl and the like and substituted carbamoyl groups such as dimethylcarbamoyl and the like.

As examples of the ether type protecting group, mention may be made of substituted silyl groups such as tert-butyldimethylsilyl, tert-butyldiphenylsilyl and the like, $(C_1-C_4)$alkoxy$(C_1-C_4)$alkyl groups such as methoxymethyl and the like, cyclic acetal groups such as tetrahydropyranyl and the like, and substituted methyl groups having one or more substituted or unsubstituted phenyl substitutents such as benzyl, 4-methoxybenzyl, trityl and the like.

As examples of the lower alkyl group of 1 to 6 carbon atoms in $R^1$, there may be mentioned straight chain alkyl groups such as methyl, ethyl, propyl and the like, and branched chain alkyl groups such as isopropyl, isobutyl and the like.

Said "physiologically acceptable salts" include alkali metal salts such as sodium salts, potassium salts and the like, alkaline earth metal salts such as calcium salts, magnesium salts and the like, ammonium salts, substituted ammonium salts, salts of mineral acids such as hydrochloric acid, sulfuric acid, nitric acid and the like, and salts of organic acids such as acetic acid, fumaric acid, maleic acid, tartaric acid,

methane-sulfonic acid and the like.

Specific examples of the compound represented by the formula (1) will be shown below. The compounds shown hereunder include all of the existing isomers, optically active isomers and racemic modifications thereof. No example of the salts is shown herein.

1. 2,6-diamino-9-[2-(1-hydroxyethyl)-3-hydroxymethyl-1-cyclobutyl]purine
2. 9-[2-(1-hydroxyethyl)-3-hydroxymethyl-1-cyclobutyl]hypoxanthine
3. 9-[2-(1-hydroxyethyl)-3-hydroxymethyl-1-cyclobutyl]adenine
4. 9-[2-(1-hydroxyethyl)-3-hydroxymethyl-1-cyclobutyl]guanine
5. 2-amino-6-chloro-9-[2-(1-hydroxyethyl)-3-hydroxymethyl-1-cyclobutyl]purine
6. 1-[2-(1-hydroxyethyl)-3-hydroxymethyl-1-cyclobutyl]cytosine
7. 1-[2-(1-hydroxyethyl)-3-hydroxymethyl-1-cyclobutyl]thymine
8. 1-[2-(1-hydroxyethyl)-3-hydroxymethyl-1-cyclobutyl]uracil
9. 5-(2-bromovinyl)-1-[2-(1-hydroxyethyl)-3-hydroxymethyl-1-cyclobutyl]uracil
10. 5-fluoro-1-[2-(1-hydroxyethyl)-3-hydroxymethyl-1-cyclobutyl]uracil
11. 9-[2-(1-hydroxypropyl)-3-hydroxymethyl-1-cyclobutyl]adenine
12. 9-[2-(1-hydroxypropyl)-3-hydroxymethyl-1-cyclobutyl]guanine
13. 2,6-diamino-9-[2-(1-hydroxypropyl)-3-hydroxymethyl-1-cyclobutyl]purine
14. 9-[2-(1-hydroxypropyl)-3-hydroxymethyl-1-cyclobutyl]hypoxanthine
15. 2-amino-6-chloro-9-[2-(1-hydroxypropyl)-3-hydroxymethyl-1-cyclobutyl]purine
16. 1-[2-(1-hydroxypropyl)-3-hydroxymethyl-1-cyclobutyl)cytosine
17. 1-[2-(1-hydroxypropyl)-3-hydroxymethyl-1-cyclobutyl]thymine
18. 1-[2-(1-hydroxypropyl)-3-hydroxymethyl-1-cyclobutyl]uracil
19. 5-(2-bromovinyl)-1-[2-(1-hydroxypropyl)-3-hydroxymethyl-1-cyclobutyl]uracil
20. 5-fluoro-1-[2-(1-hydroxypropyl)-3-hydroxymethyl-1-cyclobutyl]uracil
21. 9-[2-(1-hydroxybutyl)-3-hydroxymethyl-1-cyclobutyl]adenine
22. 9-[2-(1-hydroxybutyl)-3-hydroxymethyl-1-cyclobutyl]guanine
23. 2,6-diamino-9-[2-(1-hydroxybutyl)-3-hydroxymethyl-1-cyclobutyl]purine
24. 9-[2-(1-hydroxybutyl)-3-hydroxymethyl-1-cyclobutyl]hypoxanthine
25. 2-amino-6-chloro-9-[2-(1-hydroxybutyl)-3-hydroxymethyl-1-cyclobutyl]purine
26. 1-[2-(1-hydroxybutyl)-3-hydroxymethyl-1-cyclobutyl]cytosine
27. 1-[2-(1-hydroxybutyl)-3-hydroxymethyl-1-cyclobutyl]thymine
28. 1-[2-(1-hydroxybutyl)-3-hydroxymethyl-1-cyclobutyl]uracil
29. 5-(2-bromovinyl)-1-[2-(1-hydroxybutyl)-3-hydroxymethyl-1-cyclobutyl]uracil
30. 5-fluoro-1-[2-(1-hydroxybutyl)-3-hydroxymethyl-1-cyclobutyl]uracil

The compound of the present invention represented by the formula (1) can be obtained, for example, by treating a compound represented by the formula (4)

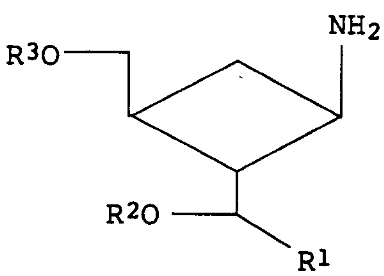

(4)

wherein $R^1$ denotes a substituted or unsubstituted lower alkyl group of 1 to 6 carbon atoms, and $R^2$ and $R^3$ each independently denote hydrogen atom or a protecting group for hydroxyl, according to a known method [D.T. Browne et al., J. Am. Chem. Soc., 90, 7302 (1968); Y.F. Shealy et al., J. Med. Chem., 30, 1090 (1987)-], for example through intermediates represented by the formula (5) and further the formula (6) as shown by the following reaction scheme (1).

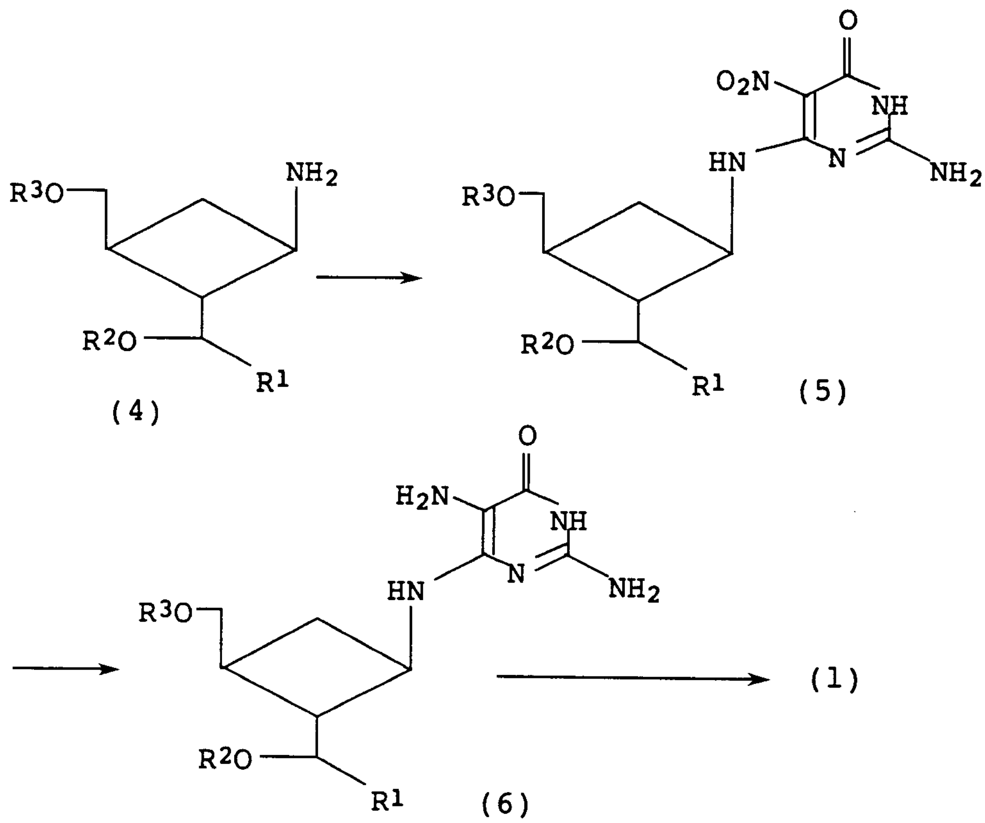

(4) (5) (6) (1)

Reaction Scheme (1)

Thus, the compound of the formula (4) is reacted with 2-amino-6-halogeno-3,4-dihydro-4-oxo-5-nitropyridine in an inert solvent such as methanol, DMF, DMSO and the like in the presence of a tertiary amine such as triethylamine and the like at temperatures from 0 ° C to near the boiling point of the solvent, preferably 20 ° C to 150 ° C, for about 1 to 12 hours to obtain the compound of the formula (5).

Then, the nitro group of the compound (5) is reduced by using a reductant such as zinc dust in an acidic solvent such as formic acid to obtain the compound of the formula (6).

The compound of the formula (6) is then reacted with any one reagent selected from formic acid, tri($C_1$-$C_4$)alkyl orthoformate such as trimethyl formate, diethoxymethoxyacetate, formamide and the like, to obtain the compound of the formula (1).

Said reagent is used in an amount of 1.0 equivalent or more, preferably 1.1 equivalent or more relative to the compound of the formula (6). It is usually employed in large excess.

These reagents may be used to serve also as a solvent. In the case of tri($C_1$-$C_4$)alkyl orthoformate, good results can be obtained when the reaction is conducted in the presence of an acid catalyst such as hydrochloric acid and the like.

When substances other than these reagents are used as a solvent, inert solvents such as DMF, DMSO, dimethylacetamide and the like may be mentioned as examples of such a solvent.

The reaction is conducted at a temperature of 0 to 190 ° C, preferably from 20 ° C to 140 ° C, for about 1 to 48 hours.

After completion of the reaction, water is added to the reaction mixture, which is then treated with aqueous ammonia solution and the like if necessary, to obtain the compound of the formula (1).

The compound represented by the formula (4), which is the starting material in the reaction scheme (1), can be prepared, for example, as shown by the following reaction scheme (2).

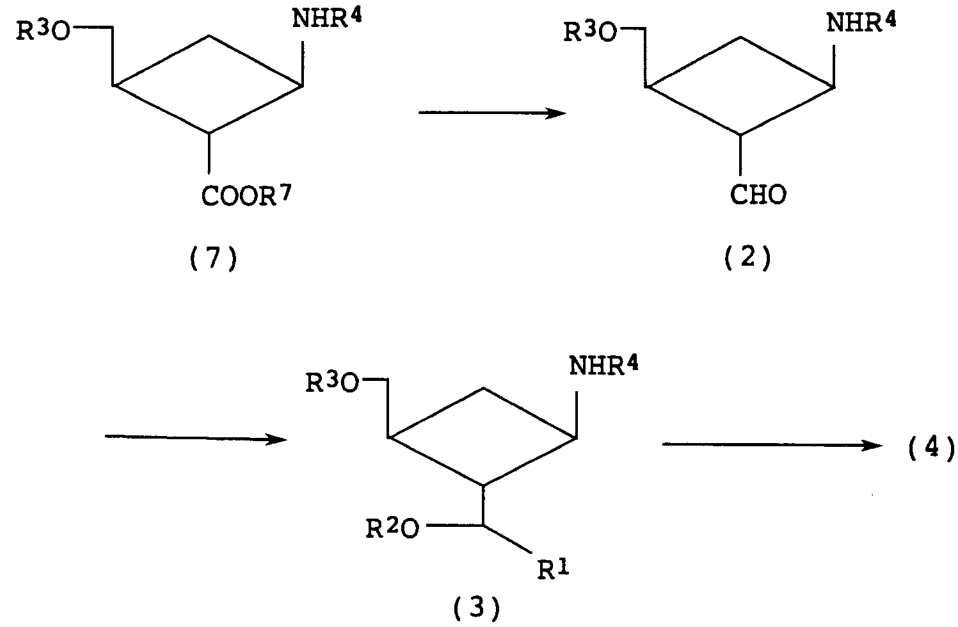

(7) (2) (3) (4)

Reaction Scheme (2)

In the reaction scheme (2), the compound represented by the formula (7), wherein $R^7$ denotes a substituted or unsubstituted straight, branched or cyclic alkoxy groups, $R^3$ denotes hydrogen atom or a protecting group for hydroxyl, and $R^4$ denotes hydrogen atom or a substituent represented by the formula -C(=X)-Y, X denoting, for example, oxygen, nitrogen or sulfur atom and Y denoting, for example, an unsubstituted or ($C_1$-$C_6$)alkyl-substituted amino group, alkyl group of 1 to 10 carbon atoms, alkoxy group of 1 to 10 carbon atoms or alkylthio group of 1 to 10 carbon atoms, can be prepared, for example, by a known method [M. Katagiri et al., Chem. Pharm. Bull., 38, 288 (1990)].

The compound represented by the formula (2) can be prepared by reducing the compound represented by the formula (7). In the reduction, any conventional reductants, for example metal hydrides, preferably diisobutylaluminum hydride (DIBAℓ-H), can be used. The reductant is used in an amount of 0.5 to 30 equivalents, preferably 1 to 10 equivalents relative to the compound of formula (7). As the solvent in the reaction are used hydrocarbons such as benzene, toluene, hexane and the like, halogenated hydrocarbons such as dichloromethane, chloroform and the like, and ethers such as tetrahydrofuran, dioxane and the like; halogenated hydrocarbons such as dichloromethane and the like give favorable results. The reaction temperature is -100 °C to 50 °C, preferably -78 °C to -30 °C, and the reaction time is 1 second to 10 hours, preferably 1 minute to 1 hour.

The compound ($R^2=H$) represented by the formula (3) can be prepared by reacting the compound represented by the formula (2) with an alkylating agent of aldehyde group, for example, $C_1$-$C_6$ alkylmetallic compound such as a Grignard reagent and the like. Specific examples of $C_1$-$C_6$ alkylmetallic compounds which may be used include lower alkylmagnesium bromide of 1 to 6 carbon atoms such as methylmagnesium bromide, ethylmagnesium bromide and the like and alkyllithium of 1 to 6 carbon atoms such as methyllithium, ethyllithium and the like. The solvents which can be used in the reaction are those which are stable to the alkylating agent, and ethereal solvents such as diethyl ether, dioxane and the like are preferable because of giving good results. The reaction is conducted at a temperature of -100 °C to 100 °C, preferably -78 °C to 50 °C.

As for the compound represented by the formula (4), when $R^4$ in the compound represented by the formula (3) is hydrogen atom the compound is equal to the compound of the formula (4). When $R^4$ is a substituent other than hydrogen atom, the compound of the formula (4) can be obtained by replacing $R^4$ with hydrogen atom by a method which may differ owing to individual substituents. For example, the compound of the formula (4) can be obtained by treatment with sodium nitrite in 10% aqueous hydrochloric acid solution when $R^4$ is carbamoyl group and by treatment with hydrochloric acid when it is tert-butoxycarbonyl group.

The optically active isomers of the compounds represented by the formula (1) can be prepared, for example, in the following manner. Thus, the compound represented by the formula (4), which is a racemic modification, is mixed with various optically active acids such as optically active carboxylic acids to obtain two kinds of diastereomer salts, from which each of the diastereomers is isolated by chromatography and crystallization or other suitable means. These diastereomers can be converted into optically active free bases represented by the formula (4) by conventional methods, for example, by decomposing the salt by addition of a base, or by using ion exchange resins. Alternatively, in a racemic compound represented by the formula (1), (2), (3), (4), (5), (6) or (7), an optically active substituent is introduced into at least one position of $R^1$, $R^2$, $R^3$, $R^4$ and $R^7$ to give diastereomers, which are then resolved to give an optically active compound. A still other method comprises the use of chemical asymmetric synthesis in the course of the preparation process. For example, an optically active compound represented by the formula (3) can be derived from a compound represented by the formula (2) by reacting therewith an organometallic reagent in the presence of a ligand capable of inducing asymmetry.

The strong antiviral activity of the compound of the present invention is demonstrated by the experimental example presented below.

Experimental Example

Antiviral activity against herpes simplex 1 virus (HSV-1), which is a DNA virus, was tested by the following method.

Vero cells (originating from kidney cells of African Green Monkey) spread in a monolayer onto 6-wells multiplate were infected with 100-150 PFU (Plaque Forming Units) of the virus. After a 1-hour adsorption period at 37°C, an agar medium (Eagle's minimum essential medium containing 1.5% agar) containing respective concentrations of the test compound (obtained in Example 4) was overlaid thereonto, and cultured in a 5% (V/V) carbon dioxide incubator at 37°C for 72 hours. The number of plaques formed was counted, from which the 50% inhibition concentration ($IC_{50}$) was determined. The results obtained are shown in the following table.

| Test Compound No. | $IC_{50}$ (μg/ml) (HSV-1) |
|---|---|
| 4-1 | 45.2 |
| 4-2 | 0.163 |

Since the compounds of the present invention represented by the formula (1) exhibit a strong antiviral activity, they are expected to be effective against herpes labialis, herpes genitalis, herpes simplex virus 1 and 2 which cause infective diseases of herpes simplex at the time of immunodepression, cytomegalo virus which causes severe pneumonia at the time of immunodepression, varicella zoster virus which is the pathogenic virus of chicken pox and herpes zoster, hepatitis A, B and C viruses, AIDS virus, and the like.

In putting the compounds of the present invention which have been obtained in the above-mentioned manner to use as antiviral agents, they can be administered orally, intravenously, percutaneously, or in other suitable ways. Their dose is usually 0.01 to 500 mg/kg/day, though it may vary depending on the infecting virus, symptom, and age of the mammal to be treated and the method of administration. The compounds of the present invention are administered in the form of a preparation produced by mixing them with an appropriate vehicle. The forms of the preparation which may be used include a tablet, granule, fine granule, powder, capsule, injection, eye drop, opthalmic ointment, ointment and suppository. The content of effective ingredients in such preparations is about 0.01 to 99.99%.

The preparation of the compound of the present invention will be illustrated in more detail by way of the following examples. All of the compounds shown herein are of racemic modification.

Example 1

Preparation of (1β,2α,3β)-1-tert-buboxycarbonylamino-3-tert-butyldimethylsiloxymethyl-2-formylcyclobutane

An anhydrous dichloromethane solution (30 ml) of (1β,2α,3β)-1-tert-buboxycarbonylamino-3-tert-butyldimethylsiloxymethyl-2-methoxycarbonylcyclobutane (1.52 g, 4.07 mmoles) was cooled to -70°C with a dry ice-acetone bath, and DIBAℓ-H (1 molar toluene solution, 6.11 ml, 11 mmoles) was added dropwise thereto. After 6 minutes stirring, 0.2 N phosphate buffer (pH 7.0) was added and the resulting mixture was brought back to room temperature with vigorous stirring. The reaction mixture was extracted 3 times with

ethyl ether, and the resulting extracts were combined, washed with water and saturated aqueous sodium chloride solution, then dried over anhydrous sodium sulfate, and the solvent was distilled off under reduced pressure. The residue was subjected to silica gel column chromatography (eluent: hexane-ethyl acetate, 5:1) to obtain (1β,2α,3β)-1-tert-butoxycarbonylamino-3-tert-butyldimethylsiloxymethyl-2-formylcyclobutane (1.40 g, 100%).

$^1$H-NMR (200 MHz, $CDCl_3$, TMS) δ(J value):
0.05 (6H, s),
0.90 (9H, s),
1.43 (9H, s),
1.78 (1H, ddd, J = 8.7, 8.7, 8.7Hz),
2.30 (1H, dd, J = 8.7, 10.6Hz),
2.41 (1H, m),
2.95 (1H, dd, J = 7.9, 2.0Hz),
3.58 (2H, dd, J = 4.0, 4.0Hz),
4.11 (1H, m),
9.81 (1H, d, J = 2.2Hz).

Example 2

Preparation of (1β,2α,3β)-1-tert-butoxycarbonylamino-3-tert-butyldimethylsiloxymethyl-2-(1-hydroxyethyl)cyclobutane

(1β,2α,3β)-1-tert-Butoxycarbonylamino-3-tert-butyldimethylsiloxymethyl-2-formylcyclobutane (1.20 g, 3.49 mmoles) was dried under reduced pressure, then dissolved in anhydrous tetrahydrofuran (15 ml) and cooled to -70 °C with a dry ice-acetone bath. To the above solution was added dropwise 0.94 mole/ℓ tetrahydrofuran solution of methylmagnesium bromide (8.90 ml, 8.38 mmoles) over a period of 6 minutes, and the reaction mixture was stirred at -70 °C for 30 minutes. After the reaction had been stopped by addition of 0.2 N phosphate buffer (pH 7.0), the reaction mixture was brought back to room temperature and extracted 3 times with ethyl ether. The extracts were combined, washed with water and aqueous saturated sodium chloride solution, then dried over anhydrous sodium sulfate, and the solvent was distilled off under reduced pressure. The residue was subjected to silica gel column chromatography (eluent:hexane-ethyl acetate, 5:1). (1β,2α,3β)-1-tert-Butoxycarbonylamino-3-tert-butyldimethylsiloxymethyl-2-formylcyclobutane, the starting material, was eluted first (386.1 mg, 32%) and then two kinds of diastereomers of (1β,2α,3β)-1-tert-butoxycarbonylamino-3-tert-butyldimethylsiloxymethyl-2-(1-hdyroxyethyl) cyclobutane were eluted in succession to obtain 185.3 mg (15.0%) and 383.8 mg (30.7%) of the diastereomers, respectively. The diasteromer eluted earlier was defined as the compound of Example 2-1 and the one eluted later was defined as the compound of Example 2-2.

$^1$H-NMR (200 MHz, $CDCl_3$, TMS) δ(J value):
(Example 2-1)
0.05 (6H, s),
0.90 (9H, s),
1.10 (3H, d, J = 6.2Hz),
1.44 (9H, s),
1.89 (2H, m),
2.24 (2H, m),
3.55 (2H, dd, J = 2.1, 4.0Hz),
3.69 (1H, m),
4.77 (1H, brd, J = 6.2Hz).
(Example 2-2)
0.03 (6H, s),
0.87 (9H, s),
1.12 (3H, d, J = 6.2Hz),
1.41 (9H, s),
1.81 (2H, m),
2.07 (1H, ddd, J = 7.3, 7.3, 7.3Hz),
2.28 (1H, ddd, J = 3.3, 7.7, 18.6Hz),
3.54 (2H, m),
3.74 (1H, m),
4.72 (1H, br).

Example 3

Preparation of (1β,2α,3β)-1-amino-2-(1-hydroxyethyl)-3-hydroxymethylcyclobutane (3-1) To an anhydrous methanol (5 ml) solution of (1β,2α,3β)-1-tert-butoxycarbonylamino-3-tert-butyldimethylsiloxymethyl-2-(1-hydroxyethyl)cyclobutane (Compound of Example 2-1, 185.3 mg, 0.54 mmole) was added 1 ml of 4N hydrochloric acid (dioxane solution) and the mixture was stirred overnight at room temperature. The solvent was distilled off from the mixture under reduced pressure, then the residue was dissolved in a small amount of methanol and neutralized by addition of sodium hydrogen carbonate. Then the precipitate was removed by filtration and the solvent was distilled off under reduced pressure. The resulting residue was subjected to silica gel column chromatography (eluent: dichloromethane-methanol, 3:1) to obtain (1β,2α,3β)-1-amino-2-(1-hydroxyethyl)-3-hydroxymethylcyclobutane-(1) (Compound of Example 3-1) (66.6 mg, 83%).
$^1$H-NMR (200 MHz, $CD_3OD$, TMS) δ(J value):
1.16 (3H, d, J = 6.3Hz),
1.75 (1H, ddd, J = 8.7, 8.7, 8.7Hz),
2.11 (2H, m),
2.33 (1H, ddd, J = 11.0, 7.7, 7.7Hz),
3.48 (1H, br),
3.55 (2H, d, J = 1.8Hz),
3.76 (1H, qD, J = 6.54, 6.5Hz).
(3-2) With 383.8 mg (1.12 mmol) of the compound of Example 2-2 used as the starting material and in the same manner as in (3-1), there was obtained (1β,2α,3β)-1-amino-2-(1-hydroxyethyl)-3-hydroxymethylcyclobutane-(2) (Compound of Example 3-2) [71.9 mg (0.5 mmol), 45%].

Example 4

Preparation of 9-[(1β,2α,3β)-2-(1-hydroxyethyl)-3-hydroxymethyl-1-cyclobutyl]guanine (Compound No. 4)
(4-1) (1β,2α,3β)-1-Amino-2-(1-hydroxyethyl)-3-hydroxymethylcyclobutane (Compound of Example 3-1, 66.6 mg, 0.46 mmole) was dissolved in anhydrous DMF (1 ml), then triethylamine (55.7 mg, 0.55 mmole) and 2-amino-6-chloro-3,4-dihydro-4-oxo-5-nitropyrimidine (87.6 mg, 0.46 mmole) were added, and the resulting mixture was warmed with an oil bath at 50 °C for 4 hours. The solvent was distilled off under reduced pressure and the residue was dissolved in formic acid (3 ml). Zinc powder (1.1 g, 18 mmoles) was added gradually to the solution over a period of 10 minutes. After completion of the addition, the mixture was filtered through Kiriyama filter paper. The zinc powder was washed with methanol, and the washing was combined with the filtrate and the solvent was distilled off from the mixture under reduced pressure to obtain 264.0 mg of crude (1β,2α,3β)-1-(2,5-diamino-3,4-dihydro-4-oxo-6-pyrimidyl)-2-(1-hydroxyethyl)-3-hydroxymethylcyclobutane. Half (132.0 mg) of the crude product was dissolved in formic acid (2.0 ml) and heated with an oil bath at 180 °C for 10 hours. After cooled by standing in air, the solution was suspended in water (2 ml), 28% aqueous ammonia solution (2 ml) was added thereto, the resulting mixture was stirred at room temperature for 2 hours and 30 minutes, and the solvent was distilled off under reduced pressure. The residue was subjected to HP-20 column chromatography (gradient elution using eluents changing from water to methanol containing 50% of water) to obtain 9-[(1β,2α,3β)-2-(1-hydroxyethyl)-3-hydroxymethyl-1-cyclobutyl]guanine-(1) (compound No. 4-1, 5.0 mg).
HPLC;
Retention time: 7.03 min
[Column: ODS-2, Solvent: 2% $CH_3CN$-98% (1% phosphate buffer), Flow rate: 1 ml/min]
$^1$H-NMR (200 MHz, $CD_3OD$, TMS) δ(J value):
1.08 (3H, d, J = 6.4Hz),
2.18 (1H, m),
2.34 (1H, m),
2.47 (1H, m),
2.67 (1H, m),
3.67 (2H, d, J = 5.1Hz),
3.86 (1H, qd, J = 6.5, 6.5Hz),
4.62 (1H, ddd, J = 8.8, 8.8, 8.8Hz),
7.88 (1H, br).
(4-2) With the compound of Example 3-2 used as the starting material and in the same manner as in (4-1), there was obtained 9-[(1β,2α,3β)-2-(1-hydroxyethyl)-3-hydroxymethyl-1-cyclobutyl]guanine-(2) (compound

No. 4-2, 15.1 mg).
HPLC;
Retention time: 4.77 min
[Column: ODS-2, Solvent: 2% $CH_3CN$-98% (1% phosphate buffer), Flow rate: 1 ml/min]
$^1$H-NMR (200 MHz, $CD_3OD$, TMS) δ(J value):
1.01 (3H, d, J = 6.3Hz),
2.19 (1H, m),
2.32 (1H, ddd, J = 9.9, 9.9, 9.9Hz),
2.52 (1H, ddd, J = 10.6, 8.1, 8.1Hz),
2.78 (1H, m),
3.68 (2H, d, J = 5.1Hz),
3.74 (1H, m),
4.53 (1H, ddd, J = 8.5, 8.5, 8.5Hz),
7.84 (1H, brs).

Further, in the same manner as in the present Example except for using trimethyl orthoformate, diethoxymethoxyacetate or formaldehyde in place of formic acid, the compound No. 4-2 is obtained.

## Claims

1. A cyclobutane derivative represented by the formula (1)

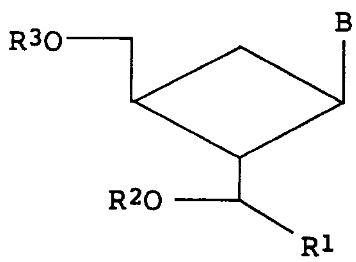

(1)

wherein B is a nucleic acid base derivative, $R^1$ denotes a lower alkyl group of 1 to 6 carbon atoms, and $R^2$ and $R^3$ each independently denote hydrogen atom or a protecting group for hydroxyl, or physiologically acceptable salts thereof.

2. A cyclobutane derivative according to claim 1, wherein B is guanine, $R^1$ is methyl, and $R^2$ and $R^3$ are both hydrogen atom.

3. A cyclobutane derivative represented by the formula (2)

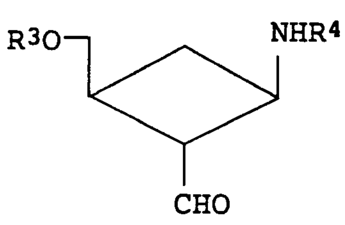

(2)

wherein $R^3$ denotes hydrogen atom or a protecting group for hydroxyl, and $R^4$ denotes hydrogen atom or a substituent.

4. A cyclobutane derivative according to claim 3, wherein $R^3$ is hydrogen atom or an ether type protecting group, $R^4$ denotes hydrogen atom or a group represented by the formula -C(=X)-Y, X denoting oxygen, nitrogen or sulfur and Y denoting an unsubstituted or ($C_1$-$C_6$)alkyl-substituted amino group, alkyl group of 1 to 10 carbon atoms, alkoxy group of 1 to 10 carbon atoms, or alkylthio group of 1 to 10

carbon atoms.

**5.** A cyclobutane derivative according to claim 3 wherein $R^3$ is t-butyldimethylsilyl group and $R^4$ is t-butoxycarbonyl group.

**6.** A cyclobutane derivative represented by the formula (3)

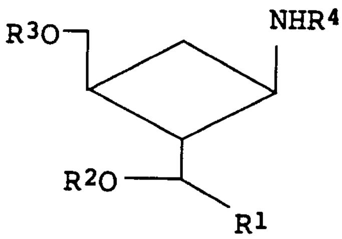

(3)

wherein $R^1$ denotes a lower alkyl group of 1 to 6 carbon atoms, $R^2$ and $R^3$ each independently denote hydrogen atom or a protecting group for hydroxyl, and $R^4$ denotes hydrogen atom or a substituent.

**7.** A cyclobutane derivative according to claim 6, wherein $R^1$ denotes methyl, $R^2$ and $R^3$ each independently denote hydrogen atom or an ether type protecting group for hydroxyl, and $R^4$ denotes hydrogen atom or a group represented by the formula -C(=X)-Y, X denoting oxygen, nitrogen or sulfur atom, Y denoting an unsubstituted or ($C_1$-$C_6$)alkyl-substituted amino group, alkyl group of 1 to 10 carbon atoms, alkoxy group of 1 to 10 carbon atoms, or alkylthio group of 1 to 10 carbon atoms.

**8.** A cyclobutane derivative according to claim 6, wherein $R^1$ is methyl, $R^2$ is hydrogen atom, $R^3$ is hydrogen atom or t-butyldimethylsilyl group, and $R^4$ is hydrogen atom or t-butoxycarbonyl group.

**9.** A pharmaceutical composition containing as an effective ingredient a cyclobutane derivative represented by the formula (1)

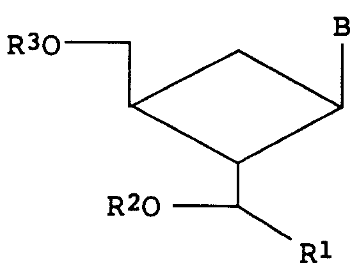

(1)

wherein B is a nucleic acid base derivative, $R^1$ denotes a lower alkyl group of 1 to 6 carbon atoms, and $R^2$ and $R^3$ each independently denote hydrogen atom or a protecting group for hydroxyl, or a physiologically acceptable salt thereof.

**10.** A composition according to claim 9, wherein B is guanine, $R^1$ is methyl group, and $R^2$ and $R^3$ are both hydrogen atom.

**11.** A composition according to claim 9 which contains 0.01-99.99% of the compound of the formula (1) based on the weight of the composition.

**12.** A method of treatment of diseases caused by herpes simplex viruses (HSV) which comprises administering to mammals infected by HSV an effective amount of a cyclobutane derivative represented by the formula (1)

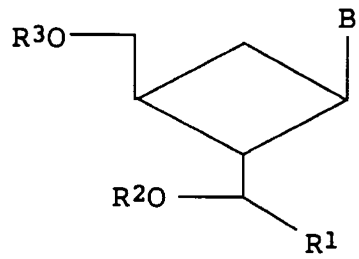 (1)

wherein B is a nucleic acid base derivative, $R^1$ denotes a lower alkyl group of 1 to 6 carbon atoms, and $R^2$ and $R^3$ each independently denote hydrogen atom or a protecting group for hydroxyl, or a physiologically acceptable salt thereof.

**13.** A method of treatment according to claim 12, wherein B is guanine, $R^1$ is methyl group, and $R^2$ and $R^3$ are both hydrogen atom.

**14.** A method of treatment according to claim 12, wherein the effective amount is 0.01-500 mg/kg/day.

**15.** Use of a cyclobutane derivative represented by the formula (1)

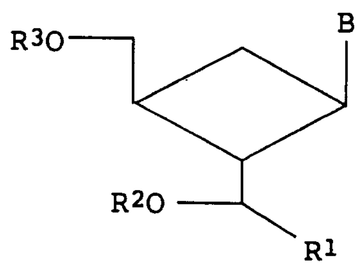 (1)

wherein B is a nucleic acid base derivative, $R^1$ denotes a lower alkyl group of 1 to 6 carbon atoms, and $R^2$ and $R^3$ each independently denote hydrogen atom or a protecting group for hydroxyl, or a physiologically acceptable salt thereof, for producing medicinal agents for treating mammals infected by herpes simplex viruses.

**16.** The use according to claim 15, wherein B is guanine, $R^1$ is methyl group, and $R^2$ and $R^3$ are both hydrogen atom.

**Claims for the following Contracting State : ES**

**1.** A process for producing a cyclobutane derivative represented by the formula (1)

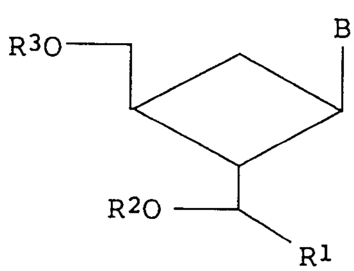 (1)

wherein B denotes guanin-9-yl, $R^1$ denotes a lower alkyl group of 1 to 6 carbon atoms, and $R^2$ and $R^3$ each independently denote hydrogen atom or a protecting group for hydroxyl, which comprises

reacting a cyclobutane derivative represented by the formula (6)

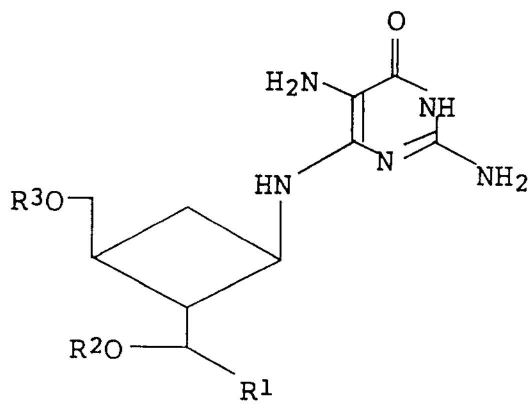

(6)

wherein $R^1$, $R^2$ and $R^3$ are as defined above, with a reagent selected from formic acid, tri($C_1$-$C_4$)alkyl orthoformates, diethoxymethoxyacetate and formamide.

**2.** A process according to claim 1, wherein $R^1$ is methyl group and $R^2$ and $R^3$ are each hydrogen atom.

**3.** A process according to claim 1, wherein the reagent is formic acid.

**4.** A process according to claim 1, wherein the reaction is conducted in a solvent at 0° to 190°C.

**5.** A process according to claim 4, wherein the solvent is formic acid.

**6.** A process according to claim 1, wherein the reaction mixture is treated with aqueous ammonia after completion of the reaction.

**7.** A process for producing a cyclobutane derivative represented by the formula (3)

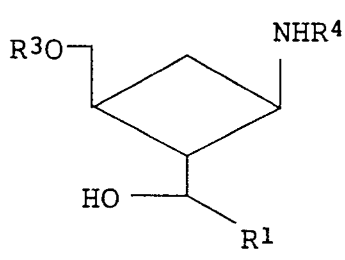

(3)

wherein $R^1$ denotes a lower alkyl group of 1-6 carbon atoms, $R^3$ denotes hydrogen atom or a protecting group for hydroxyl, and $R^4$ denotes hydrogen atom or a substituent, which comprises reacting a cyclobutane derivative represented by the formula (2)

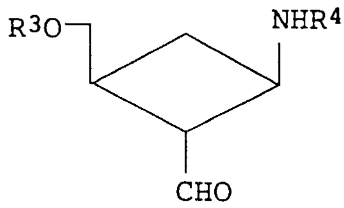

(2)

wherein $R^3$ and $R^4$ are as defined above, with an alkylating agent of aldehyde group.

**8.** A process according to Claim 7, wherein $R^1$ is a methyl group.

**9.** A process according to Claim 7, wherein the reagent is $C_1$-$C_6$ alkylmagnesium bromide or $C_1$-$C_6$ alkyllithium.

**10.** A process according to Claim 7, wherein the reaction is conducted in a solvent at -100°C to 100°C.

**11.** A process according to Claim 10, wherein the solvent is ethereal solvent.

European Patent Office

# PARTIAL EUROPEAN SEARCH REPORT

which under Rule 45 of the European Patent Convention shall be considered, for the purposes of subsequent proceedings, as the European search report

Application Number

EP 91 11 7035

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 366 059 (ABBOTT LABORATORIES)<br>* page 28 - page 29; claims 1-21 *<br>--- | 1,3,6,9,12,15 | C 07 D 473/00<br>C 07 D 239/54<br>C 07 D 239/46<br>C 07 C 223/04 |
| A | EP-A-0 335 355 (E.R. SQUIBB AND SONS, INC.)<br>* claims 1,20-22 *<br>--- | 1,3,9,12,15 | |
| A | EP-A-0 358 154 (NIPPON KAYAKU K.K.)<br>* claims 1-3,10,11 *<br>--- | 1,3,9,12,15 | |
| A,D | CHEMICAL AND PHARMACEUTICAL BULLETIN vol. 38, no. 1, 1990, pages 288 - 290; N. KATAGIRI ET AL.: 'Highly stereoselective synthesis of carbocyclic analogues of oxetanocin'<br>----- | 1,3 | |

TECHNICAL FIELDS SEARCHED (Int. Cl.5)

C 07 D
C 07 C

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

Remark : Although claims 12-14 are directed to a method of treatment of (diagnostic method practised on) the human or animal body (Article 52(4) EPC) the search has been carried out and based on the alleged effects of the compound or composition.

| Place of search | Date of completion of the search | Examiner |
|---|---|---|
| BERLIN | 16-01-1992 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0407)